(19) Europäisches Patentamt
European Patent Office
Office européen des brevets

(11) Veröffentlichungsnummer: **0 452 758 A2**

(12) **EUROPÄISCHE PATENTANMELDUNG**

(21) Anmeldenummer: **91105476.5**

(22) Anmeldetag: **06.04.91**

(51) Int. Cl.5: **E21B 43/26**, A61K 47/06, A61K 7/00

(30) Priorität: **17.04.90 DE 4012287**

(43) Veröffentlichungstag der Anmeldung: **23.10.91 Patentblatt 91/43**

(84) Benannte Vertragsstaaten: **DE FR GB IT NL**

(71) Anmelder: **CASSELLA Aktiengesellschaft**
**Hanauer Landstrasse 526**
**W-6000 Frankfurt am Main 61(DE)**

(72) Erfinder: **Engelhardt, Friedrich, Dr.**
**Hünfelder Strasse 20**
**W-6000 Frankfurt am Main 60(DE)**
Erfinder: **Ebert, Gerlinde, Dr.**
**Am Tannenstumpf 26**
**W-6072 Dreieich/Offenthal(DE)**

(74) Vertreter: **Urbach, Hans-Georg, Dr. et al**
**Hanauer Landstrasse 526**
**W-6000 Frankfurt am Main 61(DE)**

(54) **Kohlenwasserstoffreiche Gele.**

(57) Die vorliegende Erfindunge betrifft neuartige kohlenwasserstoffreiche Gele, ein Verfahren zu ihrer Herstellung und ihre Verwendung als Fracturing-Flüssigkeiten und als medizinische oder kosmetische Zubereitungen.

EP 0 452 758 A2

Die vorliegende Erfindung betrifft neuartige kohlenwasserstoffreiche Gele, ein Verfahren zu ihrer Herstellung und ihre Verwendung als Fracturing-Flüssigkeiten und als medizinische oder kosmetische Zubereitungen.

Unter einem kohlenwasserstoffreichen Gel versteht man ein System, das aus von Tensid gebildeten Polyedern besteht, die mit Kohlenwasserstoff gefüllt sind, wobei in den schmalen Zwischenräumen zwischen den Polyedern Wasser eine kontinuierliche Phase bildet. Systeme dieser Art sind bekannt und in Angew. Chem. 100 933 (1988) und Ber. Bunsenges. Phys. Chem. 92 1158 (1988) beschrieben.

Kohlenwasserstoffreiche Gele zeichnen sich durch eine Reihe rheologischer Eigenschaften aus. Beispielsweise zeigen Oszillationsexperimente die Dominanz elastischer Eigenschaften vor viskosem Verhalten, wobei die Elastizität über einen Frequenzbereich von 4 Dekaden konstant erhalten bliebt. Es zeigt sich ein Elastizitätsanstieg bei Erhöhung der Kohlenwasserstoffmenge bei gleichbleibender Menge an wäßriger Tensidlösung. Die Viskosität steigt mit ansteigendem Volumenanteil an Kohlenwasserstoff.

Kohlenwasserstoffreiche Gele zeichnen sich auch durch das Auftreten einer Fließgrenze aus. Diese Fließgrenze ist erreicht, wenn das Gel einer auferlegten Beanspruchung (Scherung, Deformation) nicht mehr standhält und zu fließen beginnt. Unterhalb der Fließgrenze weisen die Gelstrukturen Festkörpereigenschaften auf und gehorchen dem Hookeschen Gesetz. Oberhalb der Fließgrenze kommt das System im Idealfall einer newtonschen Flüssigkeit gleich.

Darüberhinaus zeigen die Systeme eine gute Thermostabilität zwischen 5 und 120°C.

Es wurde nun überraschenderweise gefunden, daß die rheologischen Eigenschaften sowie die Thermostabilität kohlenwasserstoffreicher Gele erheblich verbessert werden können, wenn die wäßrige Phase zusätzlich ein wasserlösliches Polymer enthält.

Die vorliegende Erfindung betrifft somit ein Kohlenwasserstoffreiches Gel, das aus 50 bis 99,5 Gew.% Kohlenwasserstoff, 0,005 bis 20 Gew.% Tensid und 0,49 bis 49,99 Gew.% Wasser besteht, dadurch gekennzeichnet, daß die wäßrige Phase ein wasserlösliches Polymer enthält.

Der Gewichtsanteil des wasserlöslichen Polymers beträgt bevorzugt 0,005 bis 20 Gew.%.

Ein besonders bevorzugtes erfindungsgemäßes kohlenwasserstoffreiches Gel besteht aus 70 bis 99,5 Gew.% Kohlenwasserstoff, 0,01 bis 15 Gew.% Tensid, 0,49 bis 30 Gew.% Wasser und 0,01 bis 15 Gew.% wasserlöslichem Polymer.

Ganz besonders bevorzugt ist ein erfindungsgemäßes kohlenwasserstoffreiches Gel aus 85 bis 99,3 Gew.% Kohlenwasserstoff, 0,01 bis 2 Gew.% Tensid, 0,68 bis 10 Gew.% Wasser und 0,01 bis 3 Gew.% wasserlöslichem Polymer.

Die erfindungsgemäßen kohlenwasserstoffreichen Gele enthalten als Kohlenwasserstoffkomponente beispielsweise gesättigte oder ungesättigte Aliphaten oder Aromaten. Auch Kohlenwasserstoffgemische beliebiger Mischungsverhältnisse sind geeignet.

Bevorzugte Kohlenwasserstoffe sind n-Pentan, n-Hexan, n-Heptan, n-Oktan, n-Nonan, n-Dekan, n-Dodekan, n-Tetradekan, n-Hexadekan, Cyclohexan, Cyclooktan, Benzol, Toluol, Kerosin, Benzin, bleifreies Benzin und Dieselöl.

Gegebenenfalls können die genannten Kohlenwasserstoffe aber auch durch andere Substanzen mit gleichen oder ähnlichen Eigenschaften hinsichtich der erfindungsgemäßen Systeme ersetzt werden.

So können an Stelle eines Kohlenwasserstoffs, vorteilhafterweiser wenn eine medizinische oder kosmetische Zubereitung hergestellt werden soll, auch Öle eingesetzt werden. Bei diesen Ölen handelt es sich um wasserunlösliche, bei Raumtemperatur flüssige organische Verbindungen mit relativ niedrigem Dampfdruck, wobei sowohl native als auch synthetische Verbindungen eingesetzt werden können.

Native Öle sind fast ausnahmslos Glycerinester der höheren geradzahligen Fettsäuren, die pflanzlichen oder tierischen Ursprungs sein können.

Bevorzugte tierische Öle sind insbesondere die gemischten Glyceride der Palmitin-, Stearin- und Ölsäure. Bevorzugte pflanzliche Öle sind Weizenkeimöl, Sojaöl, Avocadoöl und Kokosöl.

Öle synthetischer Herkunft sind bevorzugt flüssige Fettsäureester, flüssige Fettalkohole, niedrig-viskose Paraffinöle, Ester mehrwertiger Alkohole und Polyethylenglykole.

Besonders bevorzugte Fettsäureester sind Isopropylmyristat, Isopropylpalmitat, Isopropylstearat sowie Glyceride von Ölsäure, Laurinsäure, Myristinsäure, Palmitinsäure, Stearinsäure sowie Gemische der genannten Verbindungen.

Insbesondere für kosmetische Anwendungen finden auch Hautöle bevorzugte Anwendung. Als Hautöle werden niedrig-viskose, im allgemeinen anparfürmierte Lösungen aus flüssigen Fetten bezeichnet. Bevorzugte Bestandteile sind flüssige Fettsäureester, Silikonöle, Acetoglyceride, flüssige Wollfettderivate oder Gemische dieser Komponenten.

Die kohlenwasserstoffreichen Gele enthalten als Tensidkomponente bevorzugt Tenside mit einer Kettenlänge des aliphatischen (hydrophoben) Molekülteils von 4 bis 20 Kohlenwasserstoffatomen, wobei die

hydrophile Kopfgruppe anionisch, kationisch oder auch nichtionisch sein kann. Besonders bevorzugte Tenside sind die Verbindungen folgender Strukturen:

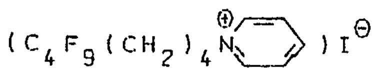

$C_5F_{11}COOH$

$C_6F_{13}CH_2COOH$

$C_8F_{17}COO^{\ominus}NH_4^{\oplus}$

$C_9F_{19}COOH$

$C_9F_{19}COO^{\ominus}NH^{\oplus}(CH_3)_3$

$C_9F_{19}COO^{\ominus}N^{\oplus}(CH_3)_4$

$C_9F_{19}COO^{\ominus}NH_4^{\oplus}$

$C_9F_{17}COO^{\ominus}NH_3^{\oplus}(C_2H_5)$

$C_9F_{17}COO^{\ominus}NH_3^{\oplus}CH_3$

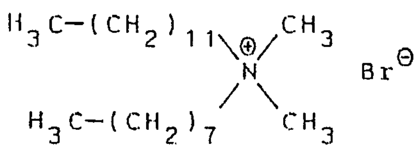

$(H_3C-(CH_2)_{11})_2-N^{\oplus}-(CH_3)_2Br^{\ominus}$

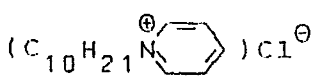

$C_{10}H_{21}SO_4^{\ominus}Na^{\oplus}$

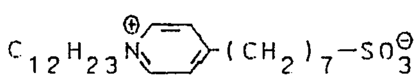

$C_{12}H_{23}NH_3^{\oplus}Cl^{\ominus}$

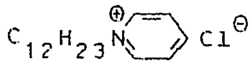

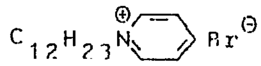

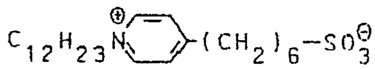

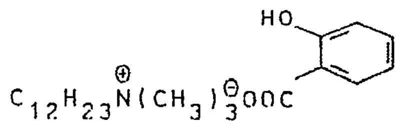

$C_{12}H_{23}\overset{\oplus}{N}(CH_3)_2-(CH_2)_8-SO_3^{\ominus}$

$C_{12}H_{23}\overset{\oplus}{N}(CH_3)_3Br^{\ominus}$

$C_{14}H_{29}\overset{\oplus}{N}(CH_3)_3Br^{\ominus}$

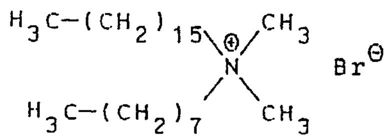

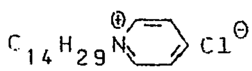

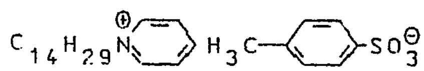

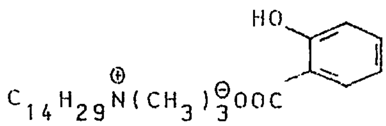

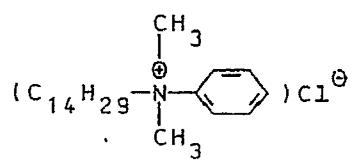

$C_{16}H_{33}\overset{\oplus}{N}(CH_3)_3Cl^{\ominus}$

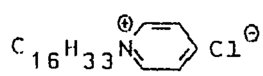

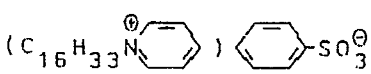

$(C_{16}H_{33}\overset{\oplus}{N}(CH_3)_3)_3PO_4^{-3}$

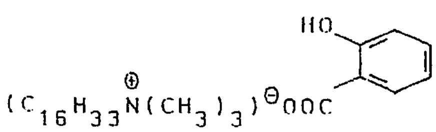

Ganz besonders bevorzugte Tenside sind

$C_{12}H_{25}OSO_3^{\ominus}Na^{\oplus}$

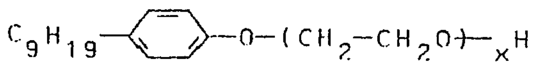

mit x = 8, 9, 10, 11, 13, 15, 23, 30 und

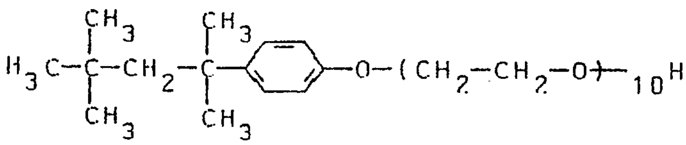

Die in der wäßrigen Phase der erfindungsgemäßen kohlenwasserstoffreichen Gele gelösten wasserlöslichen Polymere sind bevorzugt wasserlösliche Homo- und Copolymerisate auf Basis olefinisch ungesättigter Säuren, wie z.B. 2-Acrylamido-2-Methyl-propansulfonsäure, Acrylsäure, Methacrylsäure, Maleinsäure, Styrolsulfonsäure, Vinylsulfonsäure, Crotonsäure, sowie deren Salze und hydrophile Ester; hydrophile wasserlösliche Polyester, Polyvinylalkohol, wasserlösliche Cellulosederivate (z.B. Carboxymethyl-Cellulose oder Hydroxyethyl-Cellulose), sowie entsprechende Stärkederivate.

Besonders bevorzugt sind Polyacrylsäure, Polyacrylsäureamid, Poly-2-Acrylamido-2-Methyl-Propansulfonsäure, Poly-N-Vinyl-N-Methylacetamid, Polyvinylformamid, Copolymere aus Acrylsäure und Vinylacetat, Copolymere aus 2-Acrylamido2-Methyl-Propansulfonsäure, Acrylsäureamid und N-VinylN-Methyl-acetamid sowie Copolyester aus Polyethylenglykolen, Polypropylenglykolen und Terephthalsäure, Isophthalsäure und Terephthal- oder Isophthalsulfonsäuren.

Die erfindungsgemäßen kohlenwasserstoffreichen Gele können hergestellt werden, indem den nach Ber. Bunsenges. Phys. Chem. 92 1158 (1988) hergestellen Gelen die Polymere in wäßriger Lösung zugegeben werden.

Die erfindungsgemäßen kohlenwasserstoffreichen Gele weisen gegenüber den bisher bekannten Systemen erhebliche Vorteile auf, wie aus den im folgenden beschriebenen Versuchsergebnissen ersichtlich ist.

Es wurden folgende wasserlösliche Polymere untersucht:

Polymer 1: Copolymer aus Acrylsäure (80 %) und Vinylacetat (20 %)

Polymer 2: Copolymer aus 2-Acrylamido-2-Methyl-Propansulfonsäure (65 %), Acrylsäureamid (15 %) und N-Vinyl-N-Methylacetamid (20 %)

Polymer 3: Copolyester der Struktur

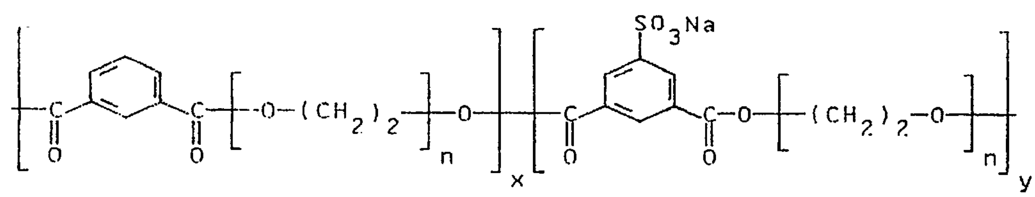

mit X = 88,75 %, Y = 11,25 %, n = 1 (80 %)und n = 2(20 %).

Polymer 4: Copolyester der Struktur

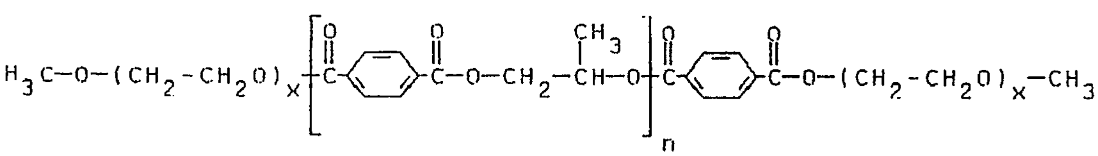

mit n = 1,25 und x = 16

Die Fließgrenze wird als die Schubspannung in der Einheit Pa ausgedrückt, bei der das Gel sich nicht mehr wie ein elastischer Festkörper verhält, sondern zu fließen beginnt. Sie wurde am CS-Rheometer von Bohlin ermittelt, wobei als Meßsystem das Platte/Platte-System (Durchmesser 2 cm, Plattenabstand 1 mm) verwendet wurde. Die Schubspannung, die durch die obere Platte an dem Gelsystem anliegt, wurde dabei schrittweise erhöht. Die Antwort des Systems, auch von der oberen Platte registriert, wurde als Schergeschwindigkeit in $sec^{-1}$ aufgezeichnet. Die Schubspannung, bei der die obere Platte zu rotieren beginnt, bei

der das Gel also der Beanspruchung nicht mehr standhält, wurde als Fließgrenze festgehalten.

Zur Messung gelangten Systeme der Zusammensetzung 0,5 g 10%ige wäßrige Natrium-Dodecylsulfat-Lösung, 40 g n-Hexan, 0,5 g X%ige wäßrige Polymerlösung (x = 0,5, 10, 20), wobei in der Tabelle 1 der Prozentgehalt an Polymerkomponente in der wäßrigen Phase angegeben ist.

Tabelle 1

| Polymer | Gehalt in (%) | Fließgrenze in (Pa) |
|---|---|---|
| 1 | 0 | 5 |
| | 2,5 | 12 |
| | 5 | 20 |
| | 10 | 70 |
| 2 | 0 | 5 |
| | 2,5 | 17 |
| | 5 | 30 |
| | 10 | 80 |
| 3 | 0 | 5 |
| | 2,5 | 15 |
| | 5 | 34 |
| | 10 | 79 |
| 4 | 0 | 5 |
| | 2,5 | 11 |
| | 5 | 24 |
| | 10 | 56 |

Der Elastizitätsmodul wurde aus Oszillationsversuchen ermittelt, in denen das Gel einer sinusförmigen Deformation ausgesetzt wurde. Die Amplitude der Deformation wurde konstant bei 0,4 % gehalten, während die Frequenz über 4 Dekaden hinweg variiert wurde. Die Ergebnisse zeigt Tabelle 2, wobei ein System aus 0,5 g 10%iger Natrium-Dodecylsulfat-Lösung, 40 g n-Hexan und 0,5 g X%iger wäßriger Polymerlösung verwendet wurde. In der Tabelle ist der prozentuale Anteil an Polymer in der wäßrigen Phase angegeben.

Tabelle 2

| Polymer | Gehalt in (%) | Elastizitätsmodul in (Pa) |
|---|---|---|
| ohne Polymerzusatz | | 75 |
| 1 | 5 | 160 |
| 2 | 2 | 220 |
| 3 | 5 | 160 |
| 4 | 5 | 130 |

Untersuchungen zur Temperaturstabilität zeigen eine erstaunliche Stabilität von Gelsystemen mit Polymerzusatz bei Temperaturen unter dem Nullpunkt. Während Systeme des Standes der Technik nur bis zu Temperaturen um 5°C stabil sind, ist bei Gelsystemen mit Polymerzusatz eine Unterkühlung bis -10°C möglich, ohne daß dabei ein Zerfall des Systems eintritt. Die bezeichnete Stabilität ist dabei weder von Gehalt oder Art des zugesetzten Polymeren anhängig.

Die erfindungsgemäßen kohlenwasserstoffreichen Gele eignen sich in hervorragender Weise als Fracturing-Flüssigkeiten bei der Tertiärförderung von Erdöl und Erdgas.

Die Gele können dabei als solche oder, bevorzugt, im Gemisch mit sogenannten Stützmitteln eingesetzt werden.

Die vorliegende Erfindung betrifft demnach auch Fracturing-Flüssigkeiten, dadurch gekennzeichnet, daß sie

a) 40 bis 99,9 Gew.% eines kohlenwasserstoffreichen Gels, das aus 50 bis 99,5 Gew.% Kohlenwasserstoff, 0,005 bis 20 Gew.% Tensid und 0,49 bis 49,99 Gew.% Wasser besteht, wobei die wäßrige Phase ein wasserlösliches Polymer enthält, und

b) 0,1 bis 60 Gew.% eines Stützmittels enthalten.

Bevorzugte Fracturing-Flüssigkeiten enthalten 45 bis 55 Gew.% kohlenwasserstoffreiches Gel und 45 bis 55 Gew.% Stützmittel.

In bevorzugten Fracturing-Flüssigkeiten enthält das kohlenwasserstoffreiche Gel 0,005 bis 20 Gew.% wasserlösliches Polymer.

In besonders bevorzugten Fracturing-Flüssigkeiten besteht das kohlenwasserstoffreiche Gel aus 70 bis 99,5 Gew.% Kohlenwasserstoff, 0,01 bis 15 Gew.% Tensid, 0,49 bis 30 Gew.% Wasser und 0,01 bis 15 Gew.% wasserlöslichem Polymer.

In ganz besonders bevorzugten Fracturing-Flüssigkeiten besteht das kohlenwasserstoffreiche Gel aus 85 bis 99,3 Gew.% Kohlenwasserstoff, 0,01 bis 2 Gew.% Tensid, 0,68 bis 10 Gew.% Wasser und 0,01 bis 3 Gew.% wasserlöslichen Polymer.

Stützmittel sind insbesondere die in der Fördertechnik üblichen und dem Fachmann bekannten Stützmittel. Bevorzugt sind Sand und Bentonite.

Stützmittel enthaltende Fracturing-Flüssigkeiten können in einfacher Weise durch Vermischen der kohlenwasserstoffreichen Gele mit der entsprechenden Menge des Stützmittels erhalten werden.

Weiterhin eignen sich die erfindungsgemäßen kohlenwasserstoffreichen Gele in hervorragender Weise als medizinische oder kosmetische Zubereitungen.

Die vorliegende Erfindung betrifft demnach auch medizinische oder kosmetische Zubereitungen, die dadurch gekennzeichnet sind, daß sie ein kohlenwasserstoffreiches Gel aus 50 bis 99,5 Gew.% Kohlenwasserstoff, 0,005 bis 20 Gew.% Tensid und 0,49 bis 49,99 Gew.% Wasser besteht, wobei die wäßrige Phase ein wasserlösliches Polymer enthält, und einen oder mehrere medizinische oder kosmetische Wirkstoffe enthalten.

In bevorzugten Zubereitungen enthält das kohlenwasserstoffreiche Gel 0,05 bis 20 Gew.% wasserlösliches Polymer.

In besonders bevorzugten Zubereitungen besteht das kohlenwasserstoffreiche Gel aus 70 bis 99,5 Gew.% Kohlenwasserstoff, 0,01 bis 15 Gew.% Tensid, 0,49 bis 30 Gew.% Wasser und 0,01 bis 15 Gew.% wasserlöslichem Polymer.

In ganz besonders bevorzugten Zubereitungen besteht das kohlenwasserstoffreiche Gel aus 85 bis 99,3 Gew.% Kohlenwasserstoff, 0,01 bis 2 Gew.% Tensid, 0,68 bis 10 Gew.% Wasser und 0,01 bis 3 Gew.%

wasserlöslichem Polymer.

Es ist besonders vorteilhaft, in den erfindungsgemäßen Zubereitungen die Kohlenwasserstoffe durch Öle, insbesondere der oben genannten Art, oder andere pharmazeutische akzeptablen Substanzen,die die gewünschte Funktion erfüllen, zu ersetzen.

Die erfindungsgemäßen Zubereitungen sind besonders zur topischen Anwendung geeignet, das heißt zum Auftragen auf Haut oder Schleimhaut.

Demnach können die Zubereitungen insbesondere alle Wirkstoffe enthalten, die über die Haut oder Schleimhaut verabreicht werden können.

Bevorzugt sind dermatologisch und/oder kosmetisch wirksame Verbindungen. Diese können synthetisch hergestellt oder Naturstoffe, zum Beispiel auch pflanzliche Extrakte, wasserlöslich oder wasserunlöslich sein, wobei wasserunlösliche Verbindungen bevorzugt sind.

Die dabei verwendeten Mengen an Wirkstoffen liegen bevorzugt zwischen 0,1 und 50 Gew.%, besonders bevorzugt zwischen 1 und 20 Gew.%.

Die erfindungsgemäßen Zubereitungen können gegebenenfalls auch übliche Hilfsmittel, beispielsweise Stabilisierungs-, Konservierungs-, Süß-, Färbe-, Geschmacks- oder Aromatisierungsmittel, enthalten.

Zur Herstellung der erfindungsgemäßen Zubereitungen können die Wirkstoffe und gegebenenfalls weitere Hilfsstoffe in einfacher Weise mit dem kohlenwasserstoffreichen Gel vermischt werden. Es ist aber auch möglich, Wirk- und Hilfsstoffe je nach Löslichkeit in der Kohlenwasserstoff- oder Wasserphase zu lösen und erst dann das kohlenwasserstoffreiche Gel herzustellen.

Beispiel 1

Bei Raumtemperatur wird eine Lösung aus 20 mg Natriumdodecylsulfat (SDS) in 170 mg Wasser vorgelegt und mittels eines Vortex Genie-Mixgerätes stark geschüttelt. Das dabei zu beobachtende starke Aufschäumen der Tensidlösung ist eine notwendige Voraussetzung für die spätere Gelbildung.

In diese stark schäumende vorgelegte Lösung werden nun - unter Fortsetzung der Schüttelbewegungen am Mixgerät - 9.56 g Pentan als Ölphase eingearbeitet. Die hydrophobe Phase wird dabei zunächst tröpfchenweise, nach erfolgter Gelbildung von ca. 3 g Gesamtmenge in größeren Mengen (ml-Mengen) auf einmal zugegeben. Ist das Mixgerät nicht mehr stark genug, die Ölphase in das hochviskose Gel einzuarbeiten, so wird die Gelherstellung durch Schüttelbewegungen mit der Hand vervollständigt.

Die Gelherstellung ist beendet, wenn der gesamte Anteil an Kohlenwasserstoff in die Gelform überführt wurde. Diesem fertigen kohlenwasserstoffreichen Gel fügt man eine Lösung aus 10 mg Polymer 2 in 240 mg $H_2O$ auf einmal zu und schüttelt das System von Hand gut durch.

Analog Beispiel 1 können die folgenden erfindungsgemäßen Gele der Beispiele 2 bis 97 hergestellt werden. Dabei werden unter den Polymeren 1, 2, 3 und 4 die bereits oben genannten Polymere verstanden.

| Beispiel | Tensid | Kohlenwasserstoff | Polymer | KW (Gew%) | Tensid (Gew%) | P (Gew%) | $H_2O$ (Gew%) |
|---|---|---|---|---|---|---|---|
| 2 | 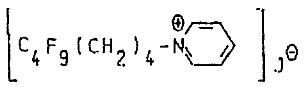 | Hexan | 3 | 83.2 | 2.1 | 0.8 | 13.9 |
| 3 | $C_5F_{11}COOH$ | Heptan | 4 | 97.9 | 0.1 | 0.3 | 1.7 |
| 4 | - " - | Oktan | 4 | 95.4 | 0.7 | 0.1 | 3.8 |
| 5 | - " - | Toluol | 1 | 72.4 | 2.1 | 0.5 | 25.0 |
| 6 | - " - | Heptan | 4 | 80.2 | 0.1 | 3.0 | 13.7 |
| 7 | $C_6F_{13}CH_2COOH$ | Nonan | 4 | 79.6 | 4.1 | 0.8 | 15.5 |
| 8 | - " - | Dekan | 2 | 90.8 | 1.2 | 0.2 | 7.8 |
| 9 | - " - | Pentan | 2 | 83.4 | 2.0 | 0.1 | 14.5 |
| 10 | - " - | Hexan | 1 | 64.8 | 0.3 | 2.4 | 32.5 |
| 11 | $C_8F_{17}COO^{\ominus}NH_4^{\oplus}$ | Cyclohexan | 4 | 63.5 | 0.2 | 6.0 | 30.3 |
| 12 | - " - | Heptan | 3 | 82.5 | 1.5 | 0.6 | 15.4 |
| 13 | - " - | Pentan | 2 | 96.25 | 0.02 | 0.02 | 3.71 |
| 14 | - " - | Oktan | 1 | 95.2 | 0.1 | 0.4 | 4.3 |
| 15 | $C_9F_{19}COOH$ | Nonan | 1 | 72.5 | 2.1 | 0.5 | 24.9 |
| 16 | - " - | Dodekan | 3 | 83.4 | 0.5 | 2.4 | 13.7 |
| 17 | - " - | Tetradekan | 3 | 63.4 | 0.7 | 4.5 | 31.4 |
| 18 | - " - | Hexadekan | 3 | 68.9 | 0.4 | 5.8 | 24.9 |
| 19 | $C_9F_{19}COO^{\ominus\oplus}NH(CH_3)_3$ | Hexan | 4 | 94.5 | 0.1 | 0.5 | 4.9 |
| 20 | - " - | Heptan | 2 | 79.4 | 0.2 | 3.0 | 17.4 |
| 21 | - " - | Oktan | 3 | 82.3 | 1.1 | 1.1 | 15.5 |
| 22 | - " - | Dekan | 3 | 84.2 | 0.9 | 2.0 | 12.9 |
| 23 | $C_9F_{19}COO^{\ominus\oplus}NH(CH_3)_4$ | Benzin | 4 | 65.2 | 0.9 | 1.8 | 32.1 |
| 24 | - " - | Hexan | 2 | 97.86 | 0.005 | 0.015 | 2.12 |
| 25 | $C_9F_{19}COO^{\ominus\oplus}NH_4$ | Nonan | 1 | 74.3 | 3.0 | 0.4 | 22.3 |
| 26 | - " - | Benzol | 3 | 82.1 | 2.1 | 0.3 | 15.5 |
| 27 | - " - | Dodekan | 4 | 79.7 | 4.1 | 0.7 | 15.5 |
| 28 | - " - | Heptan | 3 | 86.2 | 0.2 | 0.6 | 3.0 |
| 29 | $C_9F_{17}COO^{\ominus\oplus}NH_3(C_2H_5)$ | Toluol | 3 | 89.2 | 1.8 | 0.2 | 8.8 |
| 30 | - " - | Dieselöl | 4 | 76.7 | 1.2 | 1.8 | 20.3 |
| 31 | - " - | Hexan | 1 | 89.2 | 1.3 | 0.7 | 8.8 |
| 32 | - " - | Cyclohexan | 2 | 82.3 | 0.2 | 2.0 | 15.5 |
| 33 | $C_9F_{17}COO^{\ominus\oplus}NH_3CH_3$ | Heptan | 2 | 96.5 | 0.1 | 0.5 | 2.9 |

| Beispiel | Tensid | Kohlenwasserstoff | Polymer | KW (Gew%) | Tensid (Gew%) | P (Gew%) | $H_2O$ (Gew%) |
|---|---|---|---|---|---|---|---|
| 34 | - " - | Oktan | 1 | 79.6 | 4.1 | 0.8 | 15.5 |
| 35 | - " - | Hexan | 3 | 73.5 | 2.1 | 0.5 | 23.9 |
| 36 | - " - | Heptan | 3 | 79.7 | 3.9 | 0.9 | 15.5 |
| 37 | - " - | Dekan | 3 | 93.2 | 0.5 | 0.3 | 6.0 |
| 38 | 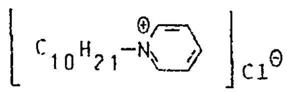 | Hexan | 2 | 98.2 | 0.1 | 0.4 | 2.1 |
| 39 | - " - | Oktan | 1 | 94.5 | 0.2 | 0.3 | 5.0 |
| 40 | - " - | Dodekan | 2 | 78.9 | 1.3 | 3.9 | 15.9 |
| 41 | - " - | Cyclooktan | 4 | 65.8 | 1.5 | 4.5 | 28.2 |
| 42 | - " - | Dekan | 3 | 69.5 | 0.2 | 7.1 | 23.2 |
| 43 | $C_{10}H_{21}SO_4^{\ominus}Na^{\oplus}$ | Benzin | 3 | 83.4 | 3.0 | 0.3 | 13.3 |
| 44 | - " - | Dodekan | 3 | 95.6 | 0.6 | 0.8 | 3.0 |
| 45 | - " - | Hexan | 1 | 89.2 | 1.8 | 0.2 | 8.8 |
| 46 | - " - | Heptan | 2 | 79.7 | 2.3 | 2.5 | 15.5 |
| 47 | - " - | Benzol | 4 | 63.4 | 2.1 | 3.1 | 31.4 |
| 48 | 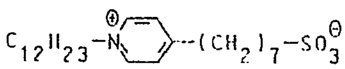 | Pentan | 3 | 61.4 | 2.9 | 4.1 | 31.6 |
| 49 | - " - | Hexan | 4 | 79.6 | 3.0 | 1.9 | 15.5 |
| 50 | - " - | Toluol | 4 | 81.0 | 2.1 | 2.8 | 14.1 |
| 51 | - " - | Dodekan | 2 | 94.5 | 0.2 | 0.4 | 4.9 |
| 52 | $C_{12}H_{23}\overset{\oplus}{N}H_3Cl^{\ominus}$ | Hexan | 1 | 93.2 | 0.1 | 0.7 | 6.0 |
| 53 | - " - | Heptan | 1 | 94.9 | 0.01 | 0.04 | 5.05 |
| 54 | - " - | Oktan | 1 | 65.2 | 1.2 | 2.6 | 31.0 |
| 55 | 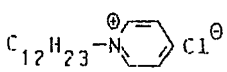 | Heptan | 3 | 74.3 | 1.1 | 2.3 | 22.3 |
| 56 | - " - | Oktan | 3 | 95.6 | 1.0 | 0.4 | 3.0 |
| 57 | - " - | Cyclooktan | 2 | 92.4 | 0.3 | 0.7 | 6.6 |
| 58 | - " - | Toluol | 4 | 79.7 | 4.8 | 0.5 | 15.0 |
| 59 | 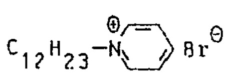 | Oktan | 1 | 84.2 | 0.015 | 0.005 | 1.94 |
| 60 | - " - | Nonan | 3 | 76.7 | 1.5 | 1.5 | 20.3 |

| Beispiel | Tensid | Kohlenwasserstoff | Polymer | KW (Gew%) | Tensid (Gew%) | P (Gew%) | $H_2O$ (Gew%) |
|---|---|---|---|---|---|---|---|
| 61 | - " - | Tetradekan | 1 | 82.5 | 0.1 | 2.0 | 15.4 |
| 62 | - " - | bleifreies Benzin | 2 | 66.3 | 1.5 | 3.3 | 28.9 |
| 63 | - " - | Aerosin | 4 | 96.5 | 0.2 | 0.4 | 2.9 |
| 64 | 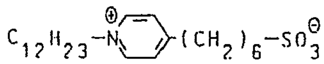 | Hexan | 3 | 82.3 | 0.3 | 1.9 | 15.5 |
| 65 | - " - | Heptan | 3 | 81.0 | 2.9 | 2.1 | 14.0 |
| 66 | - " - | Dekan | 4 | 68.9 | 0.5 | 5.5 | 25.1 |
| 67 | - " - | Dodekan | 2 | 97.8 | 0.5 | 0.5 | 1.2 |
| 68 | 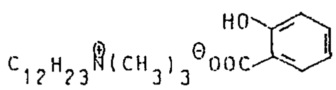 | Dodekan | 1 | 74.3 | 1.4 | 2.0 | 22.3 |
| 69 | - " - | Benzol | 2 | 82.1 | 1.0 | 0.8 | 16.1 |
| 70 | $C_{12}H_{23}\overset{\oplus}{N}(CH_3)_2-(CH_2)_8-SO_3^{\ominus}$ | Pentan | 1 | 84.5 | 1.2 | 2.0 | 12.3 |
| 71 | - " - | Dekan | 3 | 66.2 | 1.3 | 4.0 | 28.5 |
| 72 | - " - | Heptan | 3 | 86.2 | 1.3 | 1.1 | 11.4 |
| 73 | - " - | Oktan | 2 | 84.3 | 1.1 | 1.5 | 13.1 |
| 74 | $C_{12}H_{23}\overset{\oplus}{N}(CH_3)_3Br^{\ominus}$ | Dekan | 4 | 94.5 | 0.1 | 0.5 | 4.9 |
| 75 | - " - | Hexadekan | 2 | 84.8 | 1.0 | 1.9 | 12.3 |
| 76 | - " - | Hexan | 1 | 87.5 | 1.3 | 1.3 | 9.9 |
| 77 | $C_{12}H_{25}OSO_3^{\ominus}Na^{\oplus}$ | Heptan | 2 | 94.9 | 0.05 | 1.0 | 4.05 |
| 78 | - " - | Oktan | 3 | 83.5 | 1.4 | 1.5 | 13.6 |
| 79 | - " - | Hexan | 3 | 79.7 | 2.0 | 2.8 | 15.5 |
| 80 | - " - | Heptan | 3 | 97.5 | 0.4 | 0.1 | 2.0 |
| 81 | $C_{14}H_{29}\overset{\oplus}{N}(CH_3)_3Br^{\ominus}$ | Aerosin | 2 | 69.5 | 2.3 | 5.0 | 23.2 |
| 82 | - " - | Benzin | 1 | 72.5 | 1.1 | 1.5 | 24.9 |
| 83 | - " - | Hexan | 2 | 90.6 | 1.0 | 0.4 | 8.0 |
| 84 | - " - | Heptan | 1 | 82.3 | 1.2 | 1.0 | 15.5 |
| 85 | 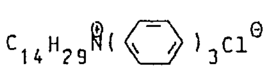 | Oktan | 4 | 74.3 | 1.5 | 1.9 | 22.3 |
| 86 | - " - | Dodekan | 2 | 83.0 | 1.1 | 2.2 | 13.7 |

| Beispiel | Tensid | Kohlenwasserstoff | Polymer | KW (Gew%) | Tensid (Gew%) | P (Gew%) | $H_2O$ (Gew%) |
|---|---|---|---|---|---|---|---|
| 87 | 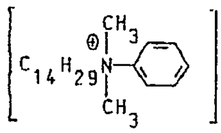 | Hexan | 1 | 72.5 | 1.4 | 1.2 | 24.9 |
| 88 | - " - | Heptan | 4 | 76.4 | 1.0 | 2.2 | 20.4 |
| 89 | $C_{16}H_{33}\overset{\oplus}{N}(CH_3)_3Cl^{\ominus}$ | Heptan | 2 | 96.2 | 0.2 | 0.6 | 3.0 |
| 90 | - " - | Oktan | 4 | 83.0 | 0.3 | 3.0 | 13.7 |
| 91 | - " - | Nonan | 4 | 82.1 | 1.4 | 1.0 | 15.5 |
| 92 | 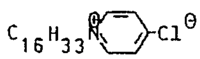 | Hexan | 2 | 96.5 | 0.1 | 0.5 | 2.9 |
| 93 | - " - | Heptan | 2 | 98.25 | 0.001 | 0.02 | 1.729 |
| 94 | - " - | Oktan | 4 | 90.5 | 0.3 | 0.9 | 8.3 |
| 95 | - " - | Dekan | 4 | 96.2 | 0.5 | 0.3 | 3.0 |
| 96 | $[C_{16}H_{33}\overset{\oplus}{N}(CH_3)_3]_3PO_4^{3-}$ | Heptan | 3 | 67.8 | 2.1 | 5.0 | 25.1 |
| 97 | - " - | Dekan | 2 | 65.2 | 1.5 | 2.3 | 31.0 |

Die folgenden Beispiele 98 bis 109 betreffen erfindungsgemäße Gele, in denen die Kohlenwasserstoffkomponente durch eine Ölphase ersetzt ist und die als kosmetische Zubereitungen oder als Grundlage für medizinische Zubereitungen verwendet werden können. Die Herstellung erfolgt analog Beispiel 1:

| Beispiel | Tensid | Kohlenwasserstoff | Polymer | KW (Gew%) | Tensid (Gew%) | P (Gew%) | $H_2O$ (Gew%) |
|---|---|---|---|---|---|---|---|
| 98 | $C_{10}H_{21}SO_4^{\ominus}Na^{\oplus}$ | Sojaöl | 2 | 90.6 | 0.6 | 0.1 | 8.7 |
| 99 | 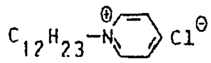 | Isopropylstearat | 1 | 95.695 | 0.1 | 0.005 | 4.2 |
| 100 | $C_{12}H_{23}\overset{\oplus}{N}H_3Cl^{\ominus}$ | Isopropylmyristat | 3 | 80.698 | 0.4 | 0.008 | 18.896 |
| 101 | 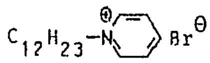 | Kokosöl | 1 | 85.48 | 0.3 | 0.02 | 14.2 |
| 102 | $C_{12}H_{23}\overset{\oplus}{N}(CH_3)_2-(CH_2)_8-SO_3^{\ominus}$ | Avocadoöl | 2 | 87.6 | 0.6 | 0.3 | 11.5 |
| 103 | $C_{12}H_{23}\overset{\oplus}{N}(CH_3)_3Br^{\ominus}$ | Isopropylpalmitat | 1 | 87.26 | 0.03 | 0.01 | 12.7 |
| 104 | $C_{12}H_{25}OSO_3^{\ominus}Na^{\oplus}$ | Sojaöl | 4 | 96.535 | 0.05 | 0.015 | 3.4 |
| 105 | $C_{14}H_{29}\overset{\oplus}{N}(CH_3)_3Br^{\ominus}$ | Weizenkeimöl | 2 | 90.7 | 0.9 | 0.9 | 8.1 |
| 106 | 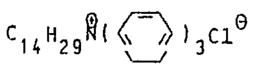 | Isopropylpalmitat | 2 | 88.85 | 0.4 | 0.05 | 10.7 |
| 107 | 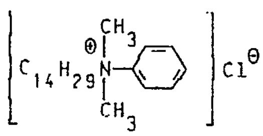 | Kokosöl | 1 | 94.88 | 0.2 | 0.07 | 4.85 |
| 108 | 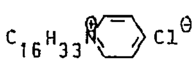 | Isopropylstearat | 1 | 92.24 | 0.6 | 0.02 | 7.14 |
| 109 | $C_{16}H_{33}\overset{\oplus}{N}(CH_3)_3Cl^{\ominus}$ | Sojaöl | 4 | 89.982 | 0.73 | 0.138 | 9.15 |

Die folgenden Beispiele 110 bis 119 betreffen erfindungsgemäße Fracturing-Flüssigkeiten. Die Herstellung erfolgt analog Beispiel 1, wobei das Stützmittel nach Bildung des Gels auf einmal zugegeben und durch Schütteln in die Gelphase eingebracht wird.

| Beispiel | Tensid | Kohlenwasserstoff | Polymer | Stützmittel | KW Gew% | Tensid Gew% | P Gew% | S Gew% | $H_2O$ Gew% |
|---|---|---|---|---|---|---|---|---|---|
| 110 | 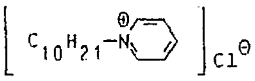 | Hexan | 2 | Montmorillionit | 66.31 | 0.8 | 0.09 | 20 | 12.8 |
| 111 | $C_{10}H_{21}SO_4^{\ominus}Na^{\oplus}$ | Heptan | 4 | Montmorillionit | 71.6 | 0.9 | 0.8 | 10 | 16.7 |
| 112 | 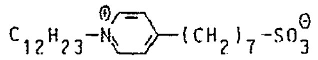 | Cyclohexan | 1 | Sand | 42.85 | 0.9 | 0.05 | 50 | 6.2 |
| 113 | $C_{12}H_{23}\overset{\oplus}{N}H_3Cl^{\ominus}$ | Heptan | 3 | Sand | 60.4 | 2.3 | 4.5 | 17 | 15.8 |
| 114 | $C_{12}H_{23}\overset{\oplus}{N}(CH_3)_3Br^{\ominus}$ | Dekan | 1 | Sand | 51.3 | 1.8 | 0.9 | 95 | 11.0 |
| 115 | $C_{14}H_{29}\overset{\oplus}{N}(CH_3)_3Br^{\ominus}$ | Oktan | 2 | Talk | 60.6 | 1.3 | 4.2 | 15 | 18.9 |
| 116 | 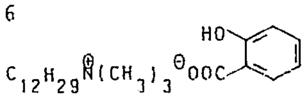 | Pentan | 2 | Kaolinit | 70.6 | 0.8 | 1.2 | 18 | 9.4 |
| 117 | 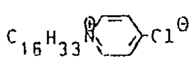 | Hexan | 4 | Sand | 62.9 | 2.3 | 4.6 | 10 | 20.2 |
| 118 | $C_{16}H_{33}\overset{\oplus}{N}(CH_3)_3Cl^{\ominus}$ | Dodekan | 1 | Talk | 69.9 | 3.8 | 2.4 | 5 | 18.5 |
| 119 | $[C_{16}H_{33}\overset{\oplus}{N}(CH_3)_3]_3PO_4^{3-}$ | Heptan | 3 | Sand | 61.51 | 0.29 | 1.3 | 31 | 5.9 |

## Patentansprüche

1. Kohlenwasserstoffreiches Gel, das aus 50 bis 99,5 Gew.% Kohlenwasserstoff, 0,005 bis 20 Gew.% Tensid und 0,49 bis 49,99 Gew.% Wasser besteht, dadurch gekennzeichnet, daß die wäßrige Phase ein wasserlösliches Polymer enthält.

2. Kohlenwasserstoffreiches Gel gemäß Anspruch 1, dadurch gekennzeichnet, daß der Gewichtsanteil des wasserlöslichen Polymers 0,005 bis 20 % beträgt.

3. Kohlenwasserstoffreiches Gel gemäß Anspruch 1 und/oder 2, dadurch gekennzeichnet, daß es aus 85 bis 99,3 Gew.% Kohlenwasserstoff, 0,01 bis 2 Gew.% Tensid, 0,68 bis 10 Gew.% Wasser und 0,01 bis 3 Gew.% wasserlöslichem Polymer besteht.

4. Kohlenwasserstoffreiches Gel gemäß einem oder mehreren der Ansprüche 1 bis 3, dadurch gekennzeichnet, daß es als Kohlenwasserstoff n-Pentan, n-Hexan, n-Heptan, n-Oktan, n-Nonan, n-Dekan, n-Dodekan, n-Tetradekan, n-Hexadekan, Cyclohexan, Cyclooktan, Benzol, Toluol, Aerosin, Benzin, bleifreies Benzin oder Dieselöl enthält.

5. Kohlenwasserstoffreiches Gel gemäß einem oder mehreren der Ansprüche 1 bis 5, dadurch gekennzeichnet, daß der Kohlenwasserstoff durch ein Öl ersetzt ist.

6. Kohlenwasserstoffreiches Gel gemäß einem oder mehreren der Ansprüche 1 bis 5, dadurch gekennzeichnet, daß es als Tensid

$C_{12}H_{25}OSO_3^{\ominus}Na^{\oplus}$

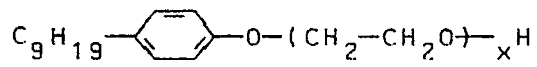

mit x = 8, 9, 10, 11, 13, 15, 23, 30 und

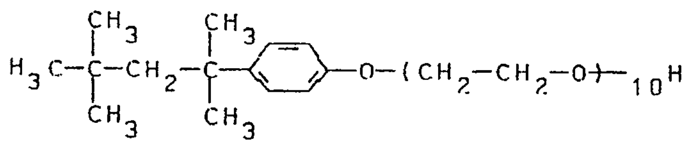

enthält.

7. Kohlenwasserstoffreiches Gel gemäß einem oder mehreren der Ansprüch 1 bis 6, dadurch gekennzeichnet, daß es als wasserlösliches Polymer Polyacrylsäure, Polyacrylsäureamid, Poly-2-Acrylamino-2-Methyl-Propansulfonsäure, Poly-N-Vinyl-N-Methylacetamid, Polyvinylformamid, Copolymere aus Acrylsäure und Vinylacetat, Copolymere aus 2-Acrylamido-2-Methyl-Propansulfonsäure, Acrylsäureamid und N-Vinyl-N-Methylacetamid oder Copolyester aus Polyethylenglykolen, Polypropylenglykolen und Terphthalsäure, Isophthalsäure und Terephthal- oder Isophthalsulfonsäuren, enthält.

8. Verwendung der kohlenwasserstoffreichen Gele gemäß einem oder mehreren der Ansprüche 1 bis 7 als Fracturing-Flüssigkeiten bei der Tertiärförderung von Erdöl und Erdgas.

9. Fracturing-Flüssigkeiten, dadurch gekennzeichnet, daß sie
   a) 40 bis 99,9 Gew.% eines kohlenwasserstoffreichen Gels, das aus 50 bis 99,5 Gew.% Kohlenwasserstoff, 0,005 bis 20 Gew.% Tensid und 0,49 bis 49,99 Gew.% Wasser besteht, wobei die wäßrige Phase ein wasserlösliches Polymer enthält, und
   b) 0,1 bis 60 Gew.% eines Stützmittels enthalten.

10. Medizinische oder kosmetische Zubereitungen, dadurch gekennzeichnet, daß sie ein kohlenwasserstoffreiches Gel aus 50 bis 99,5 Gew.% Kohlenwasserstoff, 0,005 bis 20 Gew.% Tensid und 0,49 bis 49,99 Gew.% Wasser besteht, wobei die wäßrige Phase ein wasserlösliches Polymer enthält, und einen oder mehrere medizinische oder kosmetische Wirkstoffe enthalten.